# EUROPEAN PATENT SPECIFICATION

(11) **EP 1 867 301 B1**
(45) Date of publication and mention of the grant of the patent: **23.02.2011**
(21) Application number: 07252380.6
(22) Date of filing: 12.06.2007
(51) Int. Cl.: A61F 2/38, A61F 2/36, A61L 27/18

(54) **Orthopaedic implants having bioresorbable posts**
Orthopädische Implantate mit bioresorbierbaren Stäben
Implants orthopédiques dotés de tenons biorésorbables

(30) Priority: 15.06.2006 US 453745
(43) Date of publication of application: 19.12.2007
(73) Proprietor: DePuy Products, Inc., Warsaw, IN 46581 (US)
(72) Inventor: Wyss, Joseph G., Fort Wayne, IN 46814 (US)
(74) Representative: Belcher, Simon James

(56) References cited:
- EP-A1- 0 490 616
- WO-A-2006/002476
- DE-A1-102004 034 331
- GB-A- 2 246 957
- US-A- 6 013 104

## Description

The present invention concerns orthopaedic implants and more particularly to features of such implants that help preserve the patient's natural bone.

Most joint arthroplasty procedures require replacement of one or more articulating surfaces of the joint with a prosthesis. These prosthesis are typically formed of a biocompatible metal, such as stainless steel or titanium, or occasionally of a ceramic material. The prosthesis or orthopaedic implant must be sufficiently strong to endure what may be very significant loads over a long period of time. For instance, a hip implant will endure cyclic loads which can be equivalent to as much as two or three times the patient's body weight during normal usage of the joint and even greater loads during athletic activity. Consequently, the orthopaedic implant must, by necessity, be formed of a material that can withstand these long-term loading patterns. Moreover, in some instances, the implant must provide a bearing surface that can achieve articulating motion between an opposing natural or prosthetic mating component without pitting, galling or excessive wear. Again, this requirement dictates the type of material that can be effectively used for the orthopaedic implant.

In many cases, the implant material is somewhat incompatible with simple fixation within existing bone. In other words, a porous implant that might integrate well into existing bone may not have the strength and endurance necessary to form a load-bearing component of a joint arthroplasty. Even with a bone cement interface most orthopaedic implants require an additional fixation interface with the existing bone. Thus, many orthopaedic implants rely upon lugs or posts projecting from the bone facing surface of the implant. These lugs or posts are embedded within appropriately formed bores or cavities within the natural bone and fixed, along with the bone facing surface, with bone cement or similar material.

While the use of lugs or posts results in a well-fixed implant, a significant difficulty arises during revision surgery. In general, most joint arthroplasties have a limited life. Prosthetic joint components may loosen over time. In some cases, changes in the patient's joint physiology may render a prosthesis less than optimally suited for the particular joint. In other cases, the natural bone around the implant becomes osteoporotic or may recede from the implant. Sometimes the orthopaedic implant may experience a stress failure during an excessive load event, such as a fall. Thus, many joint arthroplasties will require revision, meaning that the original joint implant is removed and replaced with a new implant component.

When the implant includes lugs or posts that extend into the natural bone, a revision surgery usually requires a large resection of the bone in order to remove the lugs/posts. This large resection not only complicates the surgery, it also requires removal of more of the patient's natural bone than is desirable. This removal of additional bone may further compromise the bone, increase the risk of onset of bone pathologies or abnormalities, or reduce the available healthy bone for fixation of the revision implant. Moreover, the large resection usually means that a larger orthopaedic implant is necessary to fill the space and restore the joint component to its expected geometry.

US-6013104 disclose a hip joint prosthesis in which the acetabular cup component has a porous bone engaging surface, and is fixed in place by means of resorbable screws.

The present invention provides an orthopaedic implant as defined in claim 1. The implant includes a post which initially augments the fixation of the implant to the prepared end of the bone.

In one aspect, the post is formed of a bioresorbable material that is selected so that its rate of resorption into the native bone is calibrated relative to the rate of bone ingrowth into the porous coating. In other words, the bioresorbable post remains viable until the bone ingrowth into the porous surface of the implant is sufficient to solidly fix the implant to the bone.

In one feature, the implant includes an engagement feature between the body and the post. In one embodiment, the engagement feature includes a threaded stem on the post and a complementary threaded feature at the bone facing surface of the body. The complementary threads may be formed in a boss projecting from the surface or in a threaded bore defined in the body, depending upon the type of implant. It is preferred that the threaded stem be formed of the same bioresorbable material as the remainder of the post.

In some embodiments, the number of fins extend continuously circumferentially around the elongated stem, while in other embodiments, the fins are discontinuous or in the form of circumferential segments. In other embodiments, the fins include a notched surface facing the direction of expulsion of the post.

It is contemplated that the features of this invention may be incorporated into a wide range of orthopaedic implants. The invention is especially valuable for surface replacement implants, such as components of a joint arthroplasty. Thus, the features of the present invention may be incorporated into a femoral, humeral or tibial surface replacement prosthesis.

The orthopaedic implant of the invention can help to simplify a potential revision surgery. More particularly, the implant can be removed in a revision surgery without requiring unnecessary removal of additional bone beneath the implant, so that the invention provides an orthopaedic implant which can be fixed securely to a prepared bone surface without the usual difficulties in a later revision procedure.

Embodiments of the invention will now be described by way of example with reference to the accompanying drawings, in which:
FIG. 1 is a bottom perspective view of a femoral surface orthopaedic implant incorporating the features of the present invention, including a bioresorbable post of the invention.
FIG. 2 is a bottom perspective view of an alternative femoral surface orthopaedic implant incorporating two bioresorbable posts.
FIG. 3 is a front perspective view of a bioresorbable post according to one embodiment of the invention, capable of use with orthopaedic implants such as the implants shown in FIGS. 1 and 2.
FIG. 4 is a front perspective view of a bioresorbable post which does not form part of the present invention, capable of use with orthopaedic implants such as the implants shown in FIGS. 1 and 2.
FIG. 5 shows a notched fin modification to the post shown in FIG. 4.
FIG. 6 is a side partial cross-sectional view of a hip surface orthopaedic implant incorporating a bioresorbable post in accordance with features of the present invention.
FIG. 7 is a side partial cross-sectional view of a humeral surface orthopaedic implant incorporating a bioresorbable post in accordance with features of the present invention.
FIG. 8 is a bottom perspective view of a tibial surface orthopaedic implant incorporating two bioresorbable posts in accordance with features of the present invention.
FIG. 9 is a cross-sectional view of an implant in accordance with one embodiment of the invention fixed within a bone and illustrating the cut line for a revision procedure to remove the surface implant.

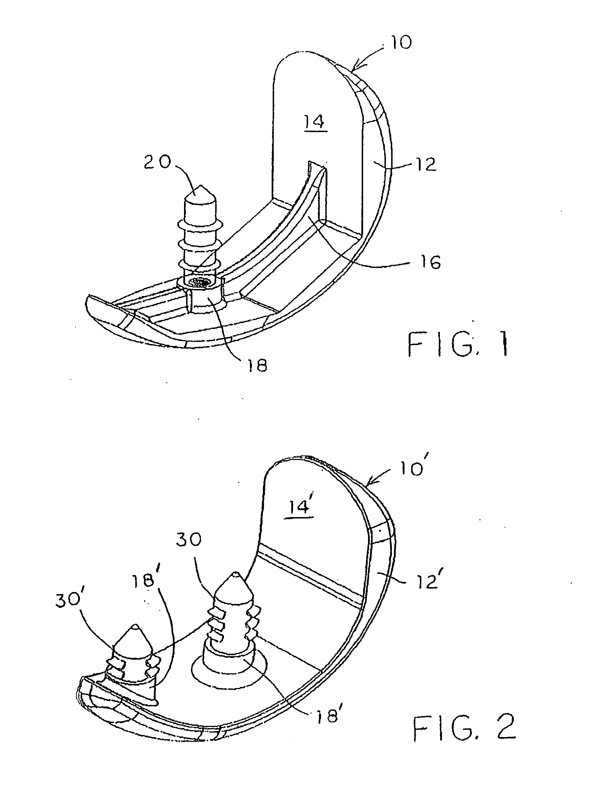

Referring to the drawings, FIG.1 shows an orthopaedic implant, specifically a femoral implant 10, which has a body 12 that is configured to replace the distal surface of the femur as part of a knee joint arthroplasty. The body is formed of a known implant material, and preferably a metallic material such as steel or titanium and alloys thereof. For some implants, the body may be formed of a biocompatible ceramic. The body 12 includes a bone facing surface 14 that is configured to mate with a distal end of a femur prepared in accordance with known practice. The bone facing surface 14 may further define a rib 16 that is positioned within a groove defined in the distal end of the femur.

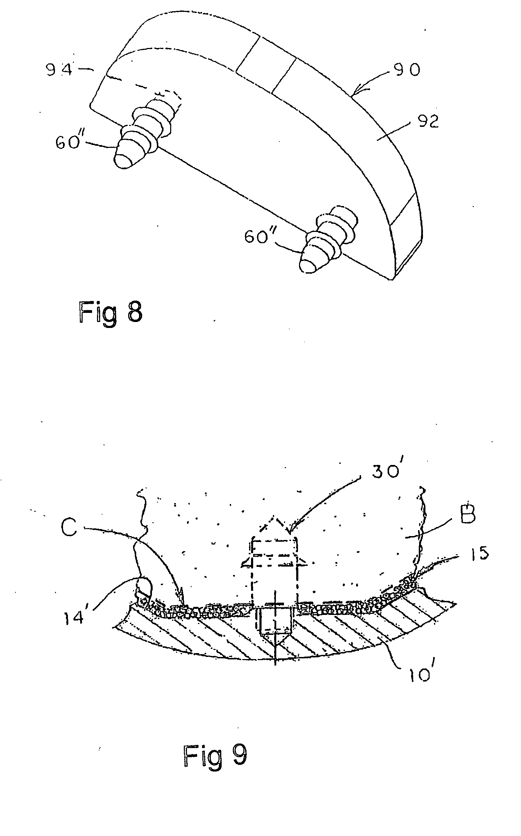

In accordance with one aspect of the invention, the facing surface 14 may be provided with a porous coating 15 (FIG. 9) that is adapted to facilitate and/or promote bone ingrowth. The porous coating enhances the fixation of the implant to the existing bone as the bony scaffolding integrates with the porous coating. The porous coating may be any suitable composition that may be affixed to, bonded to or formed on the bone facing surface 14 of the implant body 12. The bone facing surface may itself be mechanically treated to form a porous array of open cells.

In one specific embodiment, the surface 14 is coated in a known manner and to an acceptable thickness with a porous material such as that which is applied to components sold by DePuy Orthopaedics Inc under the trade mark POROCOAT. Such a coating is provided by a three-dimensional array of sintered metal alloy beads (such as cobalt-chrome or titanium). This three-dimensional array provides interconnected interstices or pores that allow bone ingrowth in and through the surface. A layer of such a coating, with a thickness from 0.76 to 0.89 mm (0.030 to 0.035 inch) and an average pore dimension of about 250 µm has been found to provide a strong implant-to-bone bond for cementless fixation of orthopaedic implants. Other types of suitable surface coatings may be applied to the implant body in a known manner, such as by plasma spraying. The porous coating for the surface 14 may be impregnated with a bone growth facilitating or encouraging substance, including certain proteins, such as bone morphogenetic proteins, demineralized bone matrix, hydroxyapatite and the like.

Even with the use of bone cement to affix the implant 10 to the prepared distal end of the femur, it is known that several weeks are required for proper fixation. In order to enhance the fixation, plugs or posts are typically provided on the bone facing surface of an orthopaedic implant. Thus, in the illustrated embodiment of FIG. 1, a post 20 is provided; however, unlike the posts of prior implants, the post 20 is formed of a bioresorbable material. The bone facing surface 14 of the implant 10 defines an engagement boss 18 that is configured to receive the post 20 is solid engagement.

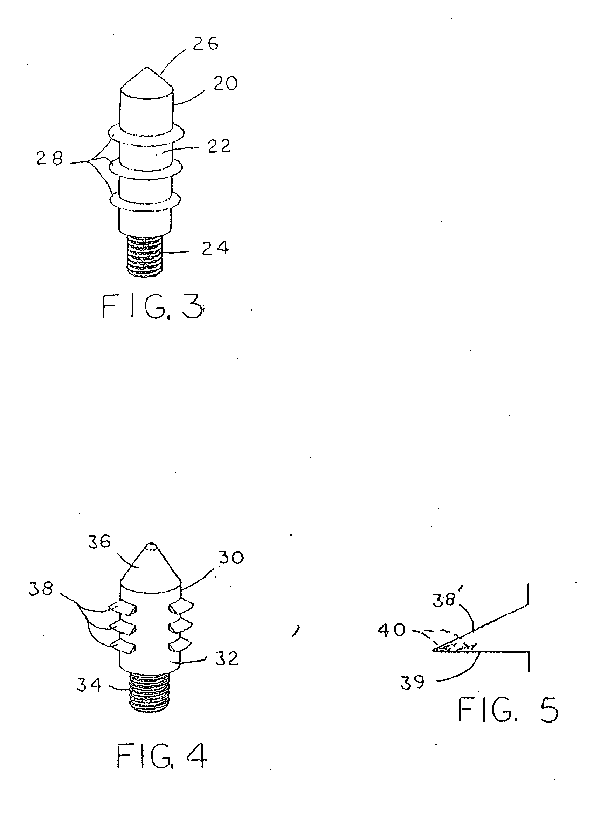

Turning to FIG. 3, one embodiment of the post 20 is illustrated. The post 20 includes an elongated stem 22 that is sized according to the bone in which the post is to be fixed. In particular, the length and diameter of the stem 22 is determined by the available underlying natural bone. The stem 22 is preferably sized in accordance with the plugs or posts incorporated into prior similar orthopaedic implants. Alternatively, the stem 22 may be smaller than the typical posts of the prior art because the porosity of the post 20 may naturally increase the effective surface are of contact between the post and the natural bone.

The bioresorbable post 20 further includes a fixation stem 24, which in the illustrated embodiment is configured with external threads. The boss 18 of the implant 10 thus includes complementary internal threads to mate with the threaded fixation stem 24. Other forms of fixation are contemplated provided that the post 20 is sufficiently rigidly fixed to the body 12 to avoid separation of the components under anticipated joint loading.

The tip 26 of the step may be tapered to facilitate introduction of the post into a prepared bore in the patient's natural bone. The tapered tip 26 is preferably configured to conform to the tapered base of the prepared bore that typically results when the bore is formed by a bone drill.

In order to enhance the fixation of the post 20 within the prepared bore, the stem 22 incorporates at least one disc-shaped fin 28 projecting outward from the surface of the stem, as shown in FIG. 3. When the implant 10 is fixed to the distal femur, this fin 28 is preferably embedded within the natural bone surrounding the prepared bore to prevent expulsion or retrograde movement of the post 20 relative to the bone. In one example which is not part of the present invention, fins 28 have a generally triangular cross-section, as represented in FIG. 5. However, other fin configurations are contemplated that permit introduction of the post into a prepared bone bore and that are able to extend outward into the bone once the post is in position.

The post 20 is formed of a bioresorbable material that is strong enough to firmly engage the natural bone and assist in retaining the implant 10 on the bone. The length of time for the post material to resorb into the adjacent bone is calibrated relative to the bone ingrowth rate into the porous coating of the bone facing surface 14 of the implant 10. In other words, the post preferably remains viable so long as the bone ingrowth is incomplete. Once the bone has fully integrated with the implant 10 through the porous coating, the structure provided by the post 20 is no longer necessary. At this time, the post may be fully resorbed into the existing natural bone.

In one aspect, the entire post 20 is formed of a resorbable material, including the engagement stem 24. Thus, when the post is fully resorbed into the existing bone, a portion of the bone will extend into the engagement boss 18. The interior of the boss may be provided with a bone ingrowth coating to enhance the fixation of the newly formed bone within the boss.

The post 20 is formed of bioresorbable materials that a known to exhibit sufficient strength in orthopaedic fixation applications. For example, suitable resorbable materials include certain polymeric materials, such as poly-alpha-hydroxy acids, poly-L-lactic acid (PLA), polyglactin acid (PGA) and derivatives or composites thereof. The post material may be impregnated with adjunct bioactive compositions to promote bone growth, healing and/or mineralization, including antibiotics, growth factors, bone morphogenetic proteins and the like. As indicated above, the selection of the bioresorbable material is preferably based on the desired resorption or degradation rate in relation to the bone ingrowth rate for the porous coating on the implant 10. Thus, the selected material may have a resorption rate measured in weeks or months, and even up to two years.

In one exemplary embodiment, the post 20 is formed of an injection moulded polymer, such as polydioxanone (PDA). The PDA material is completely resorbed in about 210 days, and is at 71 % strength in about 42 days. A typical porous coating 15 may achieve substantially complete bone ingrowth in twelve to fifteen weeks, and permanent fixation in about twenty weeks. The post may incorporate an additional resorbable layer over the PDA moulded form. For instance, a scaffold of extruded polymer fibres, such as polyglycolic acid/polylactic acid (PGA/PLA) may be fastened to the surface of the post. This PGA/PLA layer will typically have a much faster resorption rate, being fully resorbed in about ten weeks. It is further contemplated that the post 20 itself may be formed entirely of PGA/PLA where a much shorter resorption time is desired in relation to the bone ingrowth rate for the porous coating 15 of the surface implant.

In another embodiment of the invention, a femoral surface replacement implant 10' includes a body 12' that incorporates two bosses 18' on the bone facing surface 14', as shown in FIG. 2. The bosses 18' are each configured to receive a post 30. The post 30 includes an elongated stem 32 with an engagement stem 34, as shown in FIG. 4. The engagement stem 34 may incorporate threads or other forms of engagement with the complementary configured bosses 18', as in the embodiment of FIG. 3 discussed above. As with the prior embodiment, the length and diameter of the stem 32 is determined by the available bone at the prepared distal end of the femur. As illustrated in FIG. 2, one post 30 is centrally situated on the facing surface 14', while a second post 30' is offset. The second post 30' is shorter than the central post 30 because there is less available natural bone at that location to receive the post.

Returning to FIG. 4, the post 30 which does not form part of the present invention includes a tapered tip 36 and several rows of fin segments 38. The segments 38 are in lieu of the circumferential fins 28 of the post 20 in FIG. 3. The use of fin segments, rather than continuous circumferential fins, may make introduction of the post 30 into the prepared bone bore easier.

In one specific embodiment, the continuous fins 28 are essentially solid. Alternatively, as shown in FIG. 5, the fin segments 38 (and continuous fins) may incorporate notches 40 at the distal face 39 of the segments. The notches 40 face toward the direction of expulsion of the post 30 (and post 20) so that bone may be captured within the notches.

The resorbable posts of the present invention may have a range of configurations that are capable of introduction of the post into a prepared bore and that are adapted to resist expulsion or retrograde movement of the post within the natural bone.

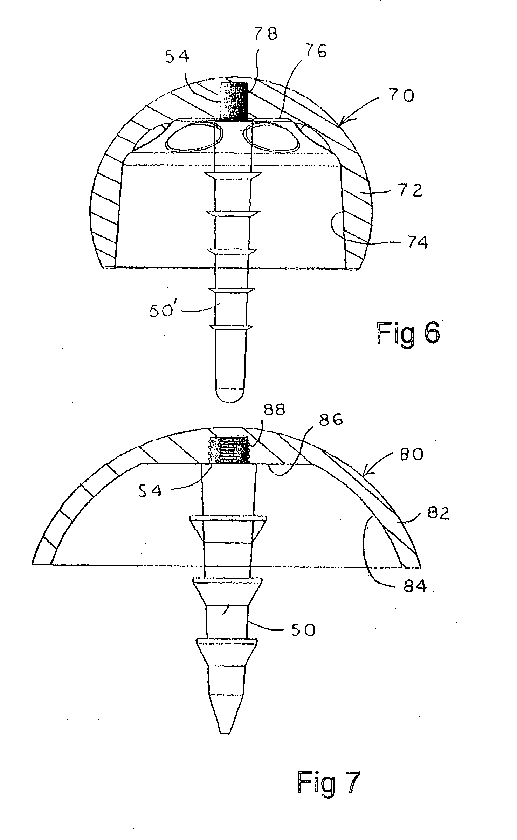

It is contemplated that the resorbable posts and porous surfaces described in connection with the femoral surface replacement implants 10 and 10' may be incorporated into other types of orthopaedic implants. Thus, as shown in FIG. 6, a hip surface replacement implant 70 includes a generally spherical cup body 72 defining a cavity 74 that conforms to the prepared proximal end of the femur as part of a hip joint arthroplasty. The proximal inner face 76 of the cup body defines a generally central engagement bore 78 to receive the engagement stem 54 of a post 50'. The length of the post 50' is such that it can extend along the intramedullary canal of the femur.

FIG. 7 shows a humeral surface replacement implant 80 which is similar in overall construction to the femoral implant. The humeral implant 80 includes a cup body 82 that defines a generally spherical cavity 84 to fit over the prepared proximal end of the humerus in a shoulder joint replacement procedure. The proximal inner face 86 defines an engagement bore 88 for receiving the engagement stem 54 of the bioresorbable post 50.

Similarly, the tibial surface replacement implant 90 shown in FIG. 8 includes a body 92 configured as a tibial tray for a knee arthroplasty. The body 92 defines a pair of engagement bores 94 for receiving the engagement stems of resorbable posts 60". It can be appreciated that the posts 60" may be similar to any of the posts 20, 30 or 60 shown in FIGS. 3 to 5.

It is contemplated that the engagement stem 24, 34 on each of the bioresorbable posts 20, 30 allows a particular post to be selected during the arthroplasty procedure. As part of the procedure, the natural bone available for receiving the implant is evaluated, and particularly the available bone for the bore or cavity prepared to receive the bioresorbable post. The length and diameter of the bore is chosen based on the available bone and the selection of available sizes of posts. Alternatively, the post could be specially fabricated to match the prepared bore or cavity.

Once the bioresorbable post has been selected it is engaged to the implant at an engagement boss, such as the bosses 18 or 18', or engagement bores, such as the bores 78 or 88. The resulting implant is then mounted to the prepared end of the bone according to the particular arthroplasty procedure.

It is contemplated that a kit may be provided in connection with a particular orthopaedic surface implant. The kit may include a selection of posts having different lengths, diameters, number and form of fins, and material resorption rates. The selection of the appropriate post(s) to be engaged to the surface implant may be determined by the nature and extent of available bone for anchoring the implant, the age and health of the patient (as it may affect rate of healing), the need for auxiliary forms of fixation such as bone cement, the type of surface implant being anchored and the composition of the porous coating on the implant.

Referring to FIG. 9, a surface implant 10' is anchored to the prepared end of a bone B. The porous coating 15 is exaggerated in the view but indicates the region of bone ingrowth between the natural bone B and the facing surface 14' of the implant. The post 30' is depicted in phantom to represent that the post has been fully resorbed into the existing bone. In a revision surgery, it can be seen that the cut line C need only follow the facing surface 14' since the post 30' is no longer viable. Thus, rather than removing a large portion of the bone B to the depth of the post 30', the only bone that must be removed is immediately adjacent the implant. Once the original implant is removed, the prepared end of the bone will require only minimal reconditioning to accept a new implant. Moreover, careful resection of the bone to remove the original implant means that the new implant can be substantially similar to the original implant, differing primarily in thickness.

The porous layers for the bone facing surfaces of each of the implants may incorporate mechanical surface features, such as grooves or "waffling". The surface features may be used to trap bone cement for fixing the implant to the prepared surface of the natural bone.

## Claims

1. An orthopaedic implant (10) for mounting on the prepared end of a bone comprising:
a body (12) having a bone facing surface (14) configured to mate with the prepared end of the bone;
a coating (15) on the bone facing surface which is porous so as to accommodate bone ingrowth when the body is mated with the bone; and
a post (20) which is formed from a bioresorbable material and extends from the bone facing surface, in use into a bore formed in the prepared end of the bone, the post including an elongate stem portion (22) having a constant diameter, **characterised in that** the stem portion has at least one disc-shaped fin (28) on it which can permit introduction of the post into a prepared bone bore and is able then to extend outwardly into the bone once the post is in position in the bore.

2. The orthopaedic implant of claim 1, which includes a plurality of the disc-shaped fins (28) on the stem portion (22), spaced apart along the stem portion.

3. The orthopaedic implant of claim 1, which includes an engagement feature (24) between the body (12) and the post (20).

4. The orthopaedic implant of claim 3, in which the engagement feature includes a threaded stem (24) on the post (20) and a complementary threaded feature at the bone facing surface (14) of the body (12).

5. The orthopaedic implant of claim 4, in which the threaded stem (24) is formed of the bioresorbable material.

6. The orthopaedic implant of claim 1, in which the fin (28) extends continuously around the elongate stem portion (22).

7. The orthopaedic implant of claim 1, in which the fin (28) has a notched surface (39) facing the direction of expulsion of the post (20).

8. The orthopaedic implant of claim 1, in which the bioresorbable material is selected so that the post (20) will not be substantially resorbed into the existing bone until substantial bone ingrowth has been achieved in the porous coating.

## Patentansprüche

1. Orthopädisches Implantat (10) zum Anbringen auf dem präparierten Ende eines Knochens, das aufweist:
einen Körper (12) mit einer zum Knochen weisenden Fläche (14), die so gestaltet ist, dass sie dem präparierten Ende des Knochens entspricht;
eine Beschichtung (15) auf der zum Knochen weisenden Fläche, die porös ist, um so Knochen einwachsen zu lassen, wenn der Körper an den Knochen angepasst ist; und
einen Zapfen (20), der aus einem bioresorbierbaren Material gebildet ist und sich von der zum Knochen weisenden Fläche erstreckt, beim Einsatz in eine Bohrung, die in dem präparierten Ende des Knochens gebildet ist, wobei der Zapfen einen länglichen Schaftbereich (22) mit einem konstanten Durchmesser umfasst, **dadurch gekennzeichnet, dass** der Schaftbereich wenigstens eine scheibenförmige Finne (28) trägt, die das Einführen des Zapfens in eine Bohrung des präparierten Knochens ermöglichen kann und dann imstande ist, sich nach außen in den Knochen zu erstrecken, sobald der Zapfen in seiner Position in der Bohrung ist.

2. Orthopädisches Implantat nach Anspruch 1, das eine Vielzahl der scheibenförmigen Finnen (28) auf dem Schaftbereich (22) umfasst, die entlang dem Schaftbereich beabstandet sind.

3. Orthopädisches Implantat nach Anspruch 1, welches ein Eingriffsmerkmal (24) zwischen dem Körper (12) und dem Zapfen (20) umfasst.

4. Orthopädisches Implantat nach Anspruch 3, bei dem das Eingriffsmerkmal einen mit Gewinde versehenen Schaft (24) auf dem Zapfen (20) und ein komplementär ausgebildetes, mit Gewinde versehenes Merkmal an der zum Knochen weisenden Fläche (14) des Körpers (12) umfasst.

5. Orthopädisches Implantat nach Anspruch 4, bei dem der mit Gewinde versehene Schaft (24) aus dem bioresorbierbaren Material gebildet ist.

6. Orthopädisches Implantat nach Anspruch 1, bei dem sich die Finne (28) kontinuierlich um den länglichen Schaftbereich (22) herum erstreckt.

7. Orthopädisches Implantat nach Anspruch 1, bei dem die Finne (28) eine gekerbte Fläche (39) hat, die der Verdrängungsrichtung des Zapfens (20) zugewandt ist.

8. Orthopädisches Implantat nach Anspruch 1, bei dem das bioresorbierbare Material so ausgewählt ist, dass der Zapfen (20) in dem vorhandenen Knochen nicht wesentlich resorbiert wird, bis wesentliches Knocheneinwachsen in die poröse Beschichtung erreicht worden ist.

## Revendications

1. Implant orthopédique (10) à monter sur l'extrémité préparée d'un os, comprenant :
✔ un corps (12) ayant une surface orientée vers l'os (14), configuré pour s'adapter avec l'extrémité préparée de l'os ;
✔ un revêtement (15) sur la surface orientée vers l'os, qui est poreux, de façon à permettre une croissance interne de l'os quand le corps est accouplé audit os ; et
✔ un montant (20) formé à partir d'un matériau biorésorbable et s'étendant depuis la surface orientée vers l'os, utilisé dans un alésage formé dans l'extrémité préparée de l'os, le montant comprenant une partie de tige allongée (22) ayant un diamètre constant, **caractérisé en ce que** la partie de tige présente au moins une ailette en forme de disque (28), qui peut permettre l'introduction du montant dans un alésage de l'os préparé et capable ensuite de s'étendre vers l'extérieur dans l'os, une fois que le montant est en position dans l'alésage.

2. Implant orthopédique selon la revendication 1, comprenant une pluralité d'ailettes en forme de disque (28) sur la partie de tige (22), espacées le long de la partie de tige.

3. Implant orthopédique selon la revendication 1, comprenant une fonction de prise (24) entre le corps (12) et le montant (20).

4. Implant orthopédique selon la revendication 3, dans lequel la fonction de prise comprend une tige filetée (24) sur le montant (20) et une fonction filetée complémentaire sur la surface orientée vers l'os (14) du corps (12).

5. Implant orthopédique selon la revendication 4, dans lequel la tige filetée (24) est formée en un matériau biorésorbable.

6. Implant orthopédique selon la revendication 1, dans lequel l'ailette (28) s'étend continuellement autour de la partie de tige allongée (22).

7. Implant orthopédique selon la revendication 1, dans lequel l'ailette (28) a une surface incisée (39) orientée dans la direction d'expulsion du montant (20).

8. Implant orthopédique selon la revendication 1, dans lequel le matériau biorésorbable est choisi de sorte que le montant (20) ne soit pas sensiblement résorbé dans l'os existant tant qu'une croissance interne osseuse sensible n'a pas été atteinte dans le revêtement poreux.
